# EUROPEAN PATENT SPECIFICATION

(11) **EP 2 807 977 B1**
(45) Date of publication and mention of the grant of the patent: **13.11.2019**
(21) Application number: 13195909.0
(22) Date of filing: 05.12.2013
(51) Int. Cl.: A61B 8/08

(54) **Ultrasound diagnosis method and aparatus using three-dimensional volume data**
Ultraschalldiagnoseverfahren und -vorrichtung mit dreidimensionalen Volumendaten
Procédé de diagnostic à ultrasons et appareil utilisant des données de volume tridimensionnel

(30) Priority: 31.05.2013 KR 20130063101
(43) Date of publication of application: 03.12.2014
(73) Proprietor: Samsung Medison Co., Ltd., Gangwon-do 250-870 (KR)
(72) Inventor: Kim, Sung-yoon, Gangwon-do (KR)
(74) Representative: Schmid, Wolfgang

(56) References cited:
- US-A1- 2005 251 036
- US-A1- 2006 189 869
- US-A1- 2007 081 705
- US-A1- 2007 249 935
- US-A1- 2009 043 202
- US-A1- 2011 028 841
- US-A1- 2012 232 394
- US-B1- 6 375 616
- US-B1- 6 575 907

## Description

### CROSS-REFERENCE TO RELATED PATENT APPLICATION

This application claims the benefit of Korean Patent Application No. 10-2013-0063101, filed on May 31, 2013, in the Korean Intellectual Property Office.

### BACKGROUND OF THE INVENTION

### 1. Field of the Invention

The present invention relates to an ultrasound diagnosis method and apparatus for diagnosing an object by using volume data displayed by an ultrasound diagnosis apparatus.

### 2. Description of the Related Art

An ultrasound diagnosis apparatus irradiates an ultrasound signal (generally, a signal having a frequency of 20 KHz or more) onto an internal specific part of an object by using a probe, and obtains an image of the internal specific part of the object by using information of an echo signal reflected from the internal specific part. Especially, the ultrasound diagnosis apparatus is used for medical purposes such as foreign material detection, injury measurement, observation, etc. of the inside of an object. The ultrasound diagnosis apparatus is more stable than X-rays, displays an image in real time, and is stable because an object is not exposed to a radioactivity. Due to such advantages, the ultrasound diagnosis apparatus is widely used along with other image diagnosis apparatuses.

An image (hereinafter referred to as an ultrasound image) obtained by the ultrasound diagnosis apparatus may be displayed by the ultrasound diagnosis apparatus, or may be stored in a storage medium and displayed by a separate image display device. For example, an ultrasound image may be reduced and displayed on a screen of a mobile terminal, a portable electronic device, a personal digital assistant (PDA), a tablet personal computer (PC), or the like.

By using the ultrasound diagnosis apparatus, a prenatal ultrasound diagnosis is performed for diagnosing a growth state of a fetus inside a pregnant woman, whether or not a fetus is a deformed fetus, or other symptoms. In the prenatal ultrasound diagnosis, a growth state of a fetus may be measured by measuring various numerical values such as a head circumference (HC), a biparietal diameter (BPD), an abdominal circumference (AC), a long bone, etc.

A length of a long bone of a fetus indicates a growth state of the fetus, and enables symptoms such as limp reduction defects to be checked. Accordingly, the length of the long bone of the fetus is used as a main indicator among measurement items of the fetus.

US 2009/043202 A1 discloses a method for producing a model of bone geometry of a volumetric region of a body. The method comprises scanning the volumetric region using ultrasound and acquiring geometric data associated with bones, identifying points derived from a surface of the bones, organizing the points into separate bone entities and transforming the points so as to position the bone entities in accordance with a normalized template. The model may be used for identifying or verifying the identity of a person.

The diagnostic ultrasound system according to US 2007/249935 A1 is provided for automatically displaying multiple planes from a volume of interest. The system comprises a transducer for acquiring ultrasound data associated with a volume of interest having a target object therein. They system further comprises a user interface for designating a reference plane within the volume on interest.

US 2011/028841 A1 discloses an ultrasound data acquisition unit configured to transmit and receive ultrasound signals to and from a target object to thereby output ultrasound data and a processing unit configured to form volume data based on the ultrasound data, set a reference slice, a reference point and a window on the volume data based on input information of a user and set a sagittal view for measuring a thickness of a nuchal translucency of a fetus on the volume data.

US 2005/251036 A1 discloses a system, method and medium for standardizing a manner of acquisition and display of ultrasound images. In one embodiment of the invention, a volumetric image of an organ is acquired in a standardized manner.

US 2006/189869 A1 discloses a system and method of creating an ultrasonic image of a hard tissue within a target. The method includes comprising transmitting from a defined location a beam energy towards the target, adjusting the angle of incidence between the beam the hard tissue to a normal angle, receiving an echo-reflection at said defined location, calculating a set of position co-ordinates the surface causing the echo-reflection repeating the sequence from additional locations, compiling position co-ordinates to generate a map of the surface causing the echo-reflection, and determining a map which represents a surface of the hard tissue by a predetermined rule.

### SUMMARY OF THE INVENTION

The related art measures a long bone of a fetus by using a two-dimensional (2D) ultrasound image that is obtained by scanning an object, in a brightness (B) mode. That is, a user should directly adjust a fan-shaped 2D ultrasound image to measure a long bone.

However, both end portions of the long bone are concave in their respective middle regions and are convex in both sides, and thus, it is difficult to measure a long bone by using the 2D ultrasound image while scanning in the B mode. Also, since each of a calf and an antebrachium has two long bones, it is easy for a user of the ultrasound diagnosis apparatus to become confused.

Due to such problems, a process that measures a long bone by using the 2D ultrasound image has a high error rate and is highly dependent on a user.

The present invention provides an ultrasound diagnosis method and apparatus using volume data.

According to an aspect of the present invention, there is provided an ultrasound diagnosis method including: deciding a reference plane for volume data acquired from an object, wherein the reference plane is obtained by cutting the volume data in a predetermined direction; detecting a long bone included in the volume data by using the reference plane; extracting long bone volume data including the long bone from the volume data; and displaying the long bone volume data.

The detecting of a long bone may include detecting the long bone based on at least one of pixel values and shapes of a plurality of areas included in the reference plane.

The ultrasound diagnosis method may further include: setting a reference point for the extracted long bone volume data; and rotating the long bone volume data with respect to the reference point such that a length axis of the long bone volume data is toward a predetermined direction.

The ultrasound diagnosis method may further include measuring a length of the detected long bone and displaying the measured length.

The reference plane may include at least one of an A plane, a B plane, and a C plane.

The extracting of long bone volume data may include extracting volume data having a predetermined thickness from a border of the detected long bone.

The object may include a pregnant woman, and the volume data may include volume data for a fetus inside the pregnant woman.

The long bone may include at least one of a humerus, a radius, an ulna, a femur, a fibula, and a tibia.

According to another aspect of the present invention, there is provided an ultrasound diagnosis apparatus including: a volume data acquirer that scans an object to acquire volume data; a reference plane decider that cuts the volume data in a predetermined direction; a long bone detector that detects a long bone included in the volume data by using the reference plane; an image processor that extracts long bone volume data including the long bone from the volume data; and a display that displays the long bone volume data.

According to another aspect of the present invention, there is provided a computer-readable record medium storing a program for executing the method.

### BRIEF DESCRIPTION OF THE DRAWINGS

The above and other features and advantages of the present invention will become more apparent by describing in detail exemplary embodiments thereof with reference to the attached drawings in which:
FIG. 1 is a diagram illustrating a body skeleton in association with an embodiment of the present invention;
FIG. 2 is a block diagram illustrating a configuration of an ultrasound diagnosis apparatus according to an embodiment of the present invention;
FIG. 3 is a flowchart illustrating an ultrasound diagnosis method according to an embodiment of the present invention;
FIG. 4 is a diagram illustrating an example in which an ultrasound image is displayed on a screen of the ultrasound diagnosis apparatus, in association with an embodiment of the present invention; and
FIG. 5 is a diagram illustrating an example in which a measured length of a long bone is displayed on a screen of the ultrasound diagnosis apparatus, in association with an embodiment of the present invention.

### DETAILED DESCRIPTION OF THE INVENTION

Terms used in the present invention have been selected as general terms which are widely used at present, in consideration of the functions of the present invention, but may be altered according to the intent of an operator skilled in the art, conventional practice, or introduction of new technology. Also, there are terms which are arbitrarily selected by the applicant in a specific case, in which case meaning of the terms will be described in detail in a corresponding description portion of the present invention. Therefore, the terms should be defined based on the entire content of this specification instead of a simple name of each of the terms.

In this disclosure below, when it is described that one comprises (or includes or has) some elements, it should be understood that it may comprise (or include or has) only those elements, or it may comprise (or include or have) other elements as well as those elements if there is no specific limitation. Moreover, each of terms such as "...unit", "...apparatus" and "module" described in specification denotes an element for performing at least one function or operation, and may be implemented in hardware, software or the combination of hardware and software.

Hereinafter, embodiments of the present invention will be described in detail with reference to the accompanying drawings.

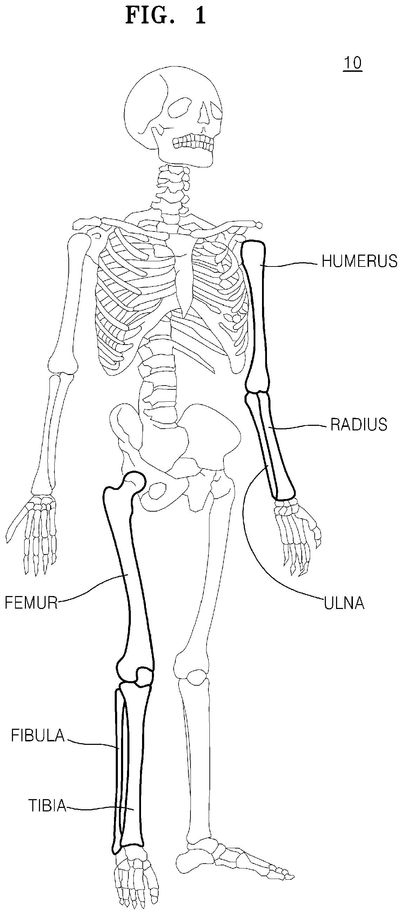

FIG. 1 is a diagram illustrating a body skeleton. A body skeleton may be largely divided into four kinds of parts such as long bones, short bones, flat bones, and irregular bones.

Among the body parts, the long bones indicate a growth state of a fetus, and thus are used as a main indicator among measurement items of the fetus. As examples of the long bones, there are a humerus, a radius, an ulna, a femur, a fibula, and a tibia, which are illustrated in FIG. 1.

In FIG. 1, the long bones constitute arms and legs, and a body part associated with of each of the long bones has a long and cylindrical shape. Also, both end portions of each of the long bones are concave in the middle and are convex on both sides.

A fetus inside a pregnant woman is not sufficiently long in length of long bones and is not clear in shape of the long bones, and for this reason, it is difficult to measure the long bones. Also, since it is unable to accurately know a pregnant position and posture, the accuracy in the determined length of each long bone depends on the expertise of a user of an ultrasound diagnosis apparatus.

Hereinafter, in order to solve such problems, an ultrasound diagnosis method and apparatus that measure a long bone by using volume data and a recording medium storing the ultrasound diagnosis method are provided.

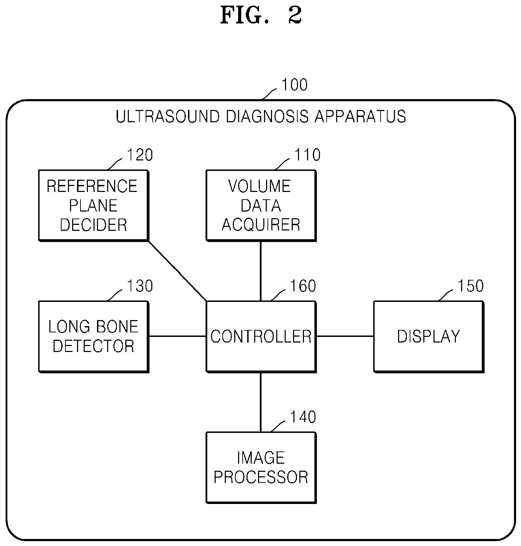

FIG. 2 is a block diagram illustrating a configuration of an ultrasound diagnosis apparatus 100 according to an embodiment of the present invention. Referring to FIG. 2, the ultrasound diagnosis apparatus 100 according to an embodiment may include a volume data acquirer 110, a reference plane decider 120, a long bone detector 130, an image processor 140, a display 150, and a controller 160. It can be understood by those skilled in the art that the ultrasound diagnosis apparatus 100 may further include another general-purpose element, in addition to the elements of FIG. 1.

The volume data acquirer 110 acquires volume data by using an ultrasound signal which is transmitted to scan an object. That is, the volume data acquirer 110 generates the volume data by receiving an echo signal generated from the object which is a response to the transmitted ultrasound signal.

A user of the ultrasound diagnosis apparatus 100 may directly acquire the ultrasound image used to generate the volume data by using a probe (not shown) included in the ultrasound diagnosis apparatus 100. Alternatively, in a picture archiving and communication system (PACS), when a ultrasound data stored in a hospital server is received by the ultrasound diagnosis apparatus 100 in a wired or wireless manner, the volume data acquirer 110 may generate volume data by using the received ultrasound data.

The volume data acquirer 110 may generate volume data from a 2D ultrasound image which is acquired by scanning an object. Alternatively, the volume data acquirer 110 may use, as volume data, a 3D image which is acquired by using a 3D probe.

The reference plane decider 120 decides a reference plane for the volume data acquired by the volume data acquirer 110. According to an embodiment, the reference plane decided by the reference plane decider 120 may include at least one of an A plane, a B plane, and a C plane which are obtained by cutting the volume data in a predetermined direction.

In the embodiment, the A plane may denote a plane which is obtained by cutting the volume data along an axial plane. Also, the B plane may denote a plane which is obtained by cutting the volume data along a sagittal plane, and the C plane may denote a plane which is obtained by cutting the volume data along a coronal plane.

Alternatively, an external input receiver (not shown) included in the ultrasound diagnosis apparatus 100 may receive an external input signal, which is used to select a direction for cutting the volume data, from a user. Therefore, the reference plane decider 120 may decide a reference plane for segmenting the volume data based on a direction which is decided by the received external input signal. In other words, the user of the ultrasound diagnosis apparatus 100 may directly decide the reference plane for cutting the volume data.

The long bone detector 130 detects a long bone from the volume data by using the reference plane. The long bone detector 130 may detect the long bone based on at least one of pixel values and shapes of a plurality of areas included in the reference plane. That is, the long bone detector 130 may detect, as an area including information on a long bone, an area having a brightness equal to or higher than a threshold value among the plurality of areas. Also, the long bone detector 130 may detect, as an area including information on a long bone, a cylinder-shaped area among the plurality of areas.

For example, when the A plane that is the reference plane includes a plurality of areas having different brightness values and shapes, the long bone detector 130 may first detect an area having a brightness equal to or higher than the threshold value. Then, the long bone detector 130 may detect an area having a cylinder-shaped area, as the area including information on the long bone. Alternatively, the long bone detector 130 may change the order of shapes and brightness values of a plurality of areas, and detect the long bone. The long bone detector 130 may detect the long bone from the volume data by using various factors in addition to the brightness values and shapes of the plurality of areas.

The image processor 140 processes the volume data in various schemes based on the detected long bone. For example, the image processor 140 may extract long bone volume data from the volume data, and set a reference point for the extracted volume data. Also, the image processor 140 may rotate the volume data, or measure a length of the extracted volume data.

The image processor 140 may include a plurality of modules that process the volume data in various schemes described above. That is, the image processor 140 may include the plurality of modules such as a volume data extraction module, a reference point setting module, a volume data rotation module, and a length measurement module.

The volume data extraction module (not shown) included in the image processor 140 extracts the long bone volume data including the detected long bone. For example, the volume data extraction module may extract long bone volume data having a predetermined thickness from a border of the detected long bone, or extract long bone volume data that includes a long bone and has a rectangular parallelepiped shape.

The reference point setting module (not shown) included in the image processor 140 may set a reference point for the extracted long bone volume data. That is, the reference point setting module may set a central point of the long bone volume data as the reference point, or set a point, corresponding to a position decided by an external input signal, as the reference point.

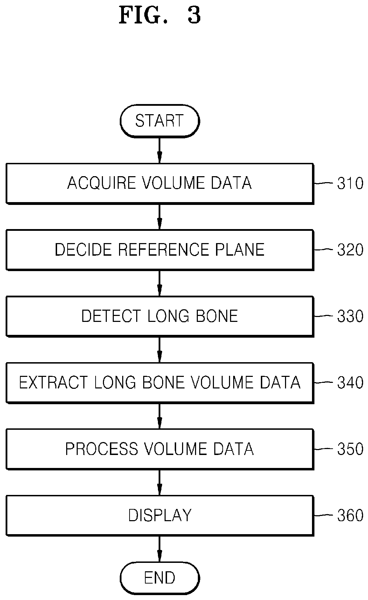

The volume data rotation module (not shown) included in the image processor 140 may rotate the volume data with respect to the reference point set by the reference point setting module. In detail, the volume data rotation module may rotate the volume data in order for a length axis of the long bone volume data to be toward a predetermined direction. Details of the embodiment will be described in detail with reference to FIG. 3.

Moreover, the length measurement module (not shown) included in the image processor 140 measures a length of the detected long bone. That is, the ultrasound diagnosis apparatus 100 may display the extracted long bone volume data, and moreover measure the length of the long bone and display the measured value to the user. Therefore, the user may immediately obtain a desired result even without additional work.

The display 150 displays at least one of the ultrasound image and the volume data. That is, the display 150 may display various ultrasound images such as the volume data acquired from the object, the reference plane which is obtained by cutting the volume data, and the extracted long bone volume data. According to an embodiment, the display 150 may display a length of the measured long bone.

The display 150 may include at least one of a liquid crystal display, a thin film transistor-liquid crystal display, an organic light emitting diode display, a flexible display, and a 3D display. Also, the ultrasound diagnosis apparatus 100 may include two or more displays 150 depending on an implementation type.

According to an embodiment, the display 150 may include the external input receiver (not shown) and a touch screen having a layer structure. That is, the display 150 may be used as both an output device and an input device, in which case the display 150 may receive a touch input using a stylus pen or a part of a human body.

Moreover, as described above, when the display 150 has the layer structure and includes the touch screen, the display 150 may detect a touch input position, a touch area, and a touch pressure. Also, the touch screen may detect a proximity touch as well as a real touch.

The controller 160 controls an overall operation of the elements included in the ultrasound diagnosis apparatus 100. That is, the controller 160 controls the reference plane decider 120 such that the volume data acquirer 110 decides the reference plane for the acquired volume data. Also, the controller 160 may control the long bone detector 130 so as to detect the long bone from the reference plane decided by the reference plane decider 120, or control the display 150 so as to display an ultrasound image.

The ultrasound diagnosis apparatus 100 may further include a storage device (not shown) in addition to the illustrated elements. The storage device may store an ultrasound image which is acquired by the ultrasound diagnosis apparatus 100 scanning an object. Alternatively, the storage device may store an ultrasound image which is received from the external server in the PACS. The storage device may store an image of the detected long bone in addition to the above-described details, or store the volume data processed by the image processor 140.

FIG. 3 is a flowchart illustrating an ultrasound diagnosis method according to an embodiment of the present invention. Referring to FIG. 3, the ultrasound diagnosis method includes a plurality of operations which are sequentially performed by the volume data acquirer 110, reference plane decider 120, long bone detector 130, image processor 140, display 150, and controller 160 of the ultrasound diagnosis apparatus 100. Therefore, despite details which are not described below, the above-described details of the elements of FIG. 2 may be applied to a description of the flowchart of FIG. 3.

In operation 310, the ultrasound diagnosis apparatus 100 acquires volume data. The ultrasound diagnosis apparatus 100 may generate the volume data by using an ultrasound data which is acquired by scanning the body of a pregnant woman, or receive an ultrasound data (which is previously obtained and stored in the external server) in a wired or wireless manner to generate the volume data. Also, the ultrasound diagnosis apparatus 100 may measure the body of the pregnant woman by using the 3D probe to directly acquire the volume data.

In operation 320, the ultrasound diagnosis apparatus 100 decides a reference plane for the volume data. That is, the ultrasound diagnosis apparatus 100 may decide on at least one reference plane by cutting the volume data in a predetermined direction.

For example, when the ultrasound diagnosis apparatus 100 generates volume data, the ultrasound diagnosis apparatus 100 may decide a plane, selected from among a plurality of ultrasound images, as a reference plane for each of an A plane, a B plane, and a C plane. Alternatively, the ultrasound diagnosis apparatus 100 may decide the reference plane based on an external input signal received from a user.

In operation 330, the ultrasound diagnosis apparatus 100 detects a long bone by using the reference plane. That is, the ultrasound diagnosis apparatus 100 may detect an area, including information on the long bone, from among a plurality of areas included in the reference plane.

The ultrasound diagnosis apparatus 100 may detect the long bone based on at least one of brightness values and shapes of a plurality of areas. As described above, a brightness value of an area corresponding to a bone is shown higher than that of an area corresponding to a tissue, and thus, the ultrasound diagnosis apparatus 100 may detect, as area including information on a long bone, an area having a brightness value equal to or higher than a threshold value.

Alternatively, the ultrasound diagnosis apparatus 100 may detect, as area including information on a long bone, an area having a cylindrical shape and corresponding to the long bone from among a plurality of areas having a brightness value equal to or higher than the threshold value. The ultrasound diagnosis apparatus 100 may detect the long bone by using various references and algorithms in addition to a brightness value and a shape.

In operation 340, the ultrasound diagnosis apparatus 100 extracts long bone volume data from the volume data which has been acquired in operation 310. That is, the ultrasound diagnosis apparatus 100 extracts the long bone volume data by using the information on the long bone detected from the reference plane.

In operation 340, the ultrasound diagnosis apparatus 100 may extract, as the long bone volume data, volume data having a certain size and including the long bone which has been detected in operation 330. For example, the ultrasound diagnosis apparatus 100 may detect, as the long bone volume data, volume data having a predetermined uniform thickness from a border of the detected long bone. Alternatively, the ultrasound diagnosis apparatus 100 may decide volume data having a rectangular parallelepiped shape and including the detected long bone, and detect, as the long bone volume data, the decided volume data having a rectangular parallelepiped shape. In other words, in operation 340, the ultrasound diagnosis apparatus 100 may detect, as the long bone volume data, volume data having various shapes and including the detected long bone.

In operation 350, the ultrasound diagnosis apparatus 100 processes the volume data in various schemes. As a first example, the ultrasound diagnosis apparatus 100 may decide on a reference point for the long bone volume data. That is, the ultrasound diagnosis apparatus 100 may decide a point, which is a spatial center, as a reference point in the extracted long bone volume data.

As a second example, in operation 350, the ultrasound diagnosis apparatus 100 may rotate the volume data with respect to the reference point. That is, before displaying the volume data to the user, the ultrasound diagnosis apparatus 100 may rotate entire volume data in order for the long bone volume data to be toward a predetermined direction.

In deciding a degree by which the volume data is rotated, the ultrasound diagnosis apparatus 100 may rotate the volume data in order for a length axis of the detected long bone to be toward the predetermine direction. That is, the ultrasound diagnosis apparatus 100 may rotate the volume data so as to be aligned in a vertical direction or a lateral direction on a screen of the ultrasound diagnosis apparatus 100.

As a third example, in operation 360, the ultrasound diagnosis apparatus 100 may measure a length of the detected long bone. The ultrasound diagnosis apparatus 100 may display the detected long bone volume data to the user, and simultaneously measure the length of the long bone and display the measured length to the user. In operation 360, the ultrasound diagnosis apparatus 100 processes the volume data through at least one of the above-described examples.

In operation 370, the ultrasound diagnosis apparatus 100 displays the long bone volume data. That is, the ultrasound diagnosis apparatus 100 displays the processed volume data and/or long bone volume data. According to an embodiment, when the ultrasound diagnosis apparatus 100 has measured the length of the detected long bone, the ultrasound diagnosis apparatus 100 may display the measured length along with the volume data.

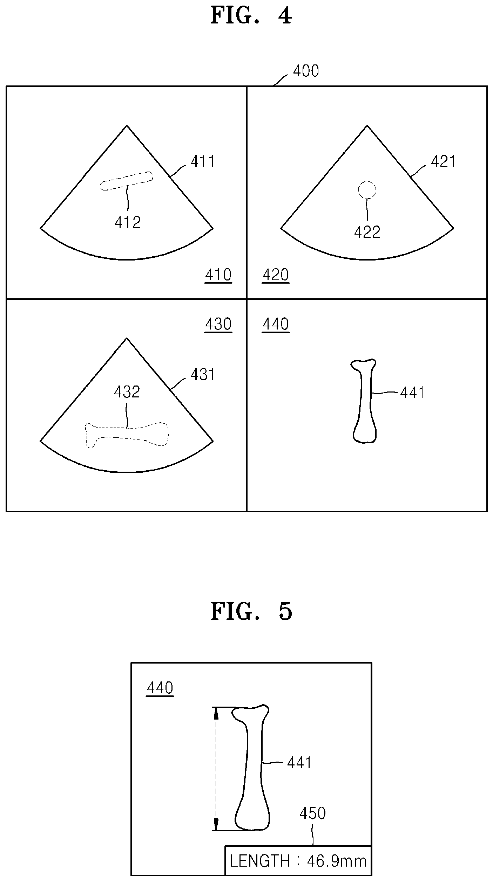

FIG. 4 is a diagram illustrating an example in which an ultrasound image is displayed on a screen 400 of the ultrasound diagnosis apparatus 100, in association with an embodiment of the present invention.

The ultrasound diagnosis apparatus 100 divides the screen 400 into a plurality of areas 410, 420, 430 and 440, and displays a plurality of reference planes 411, 421 and 431 for segmenting acquired volume data and in the plurality of areas 410, 420 and 430, respectively. That is, the ultrasound diagnosis apparatus 100 may display a first reference plane (the A plane 411) in the first area 410, display a second reference plane (the B plane 421) in the second area 420, and display a third reference plane (the C plane) in the third area 430.

Subsequently, the ultrasound diagnosis apparatus 100 detects long bones 412, 422 and 432 from the respective reference planes 411, 421 and 431. A long bone has a cylindrical shape, and is shown in different shapes and in the reference planes 411, 421 and 431. As illustrated in FIG. 4, the long bone 412 may be shown as having a cylindrical shape in the A reference plane 411, the long bone 422 may be shown as having a circular shape in the B reference plane 421, and the long bone 432 may be shown as having a bony shape in the C reference plane 431.

Therefore, the ultrasound diagnosis apparatus 100 may first detect an area, including information on a long bone, from one of the reference planes 411, 421 and 431 based on a brightness value and a shape. Subsequently, the ultrasound diagnosis apparatus 100 may compare a brightness value and shape of an area (which is detected from among different reference planes) and a brightness value and shape of a corresponding area to check for the presence of a long bone.

For example, the ultrasound diagnosis apparatus 100 may decide, as an area including information on a long bone, the area 412 having a brightness value equal to or higher than the threshold value and a cylindrical shape from among a plurality of areas included in the A reference plane 411. Subsequently, the ultrasound diagnosis apparatus 100 may detect a brightness value and shape of the area 422 corresponding to the area 412 among a plurality of areas included in the B reference plane 421, and determine whether the detected brightness value is equal to or higher than the threshold value. Likewise, the ultrasound diagnosis apparatus 100 may check whether the detected area represents a long bone by using a shape and brightness value of the area 432 corresponding to the area 412 among a plurality of areas included in the C reference plane 431. Through such operations, the ultrasound diagnosis apparatus 100 can increase a reliability of information on the detected long bone.

Subsequently, the ultrasound diagnosis apparatus 100 extracts long bone volume data 441 from acquired volume data based on the detected long bone. An operation of extracting the long bone volume data 441 is as described above with reference to FIGS. 2 and 3. In FIG. 4, an embodiment of the long bone volume data 441 having a predetermined uniform thickness from a border of the detected long bone is illustrated.

The ultrasound diagnosis apparatus 100 may extract long bone volume data having a rectangular parallelepiped shape and including the detected long bone, or extract long bone volume data having various shapes.

Various processing operations being performed on volume data before the ultrasound diagnosis apparatus 100 displays the long bone volume data 441 is as described above. That is, the ultrasound diagnosis apparatus 100 may decide a spatial center point of the detected long bone as a reference point, and rotate the volume data with respect to the reference point. In FIG. 4, an embodiment in which a length axis of the long bone volume data 441 is aligned in a vertical direction through the operation is illustrated.

Alternatively, the ultrasound diagnosis apparatus 100 may project the long bone volume data 441 on a predetermined plane, or perform a 3D rendering operation on the volume data, thus providing a high-quality ultrasound image to a user.

Subsequently, the ultrasound diagnosis apparatus 100 may display the extracted and processed long bone volume data 441 in the fourth area 440 of the screen 400.

According to the embodiment described above with reference to FIG. 4, the user may simultaneously observe the plurality of reference planes 411, 421 and 431, which are obtained by cutting the volume data in various directions, and the long bone volume data 441.

According to the embodiment, a user can accurately determine a position of the long bone volume data 441 in entire volume data, thus efficiently diagnosing an object. Also, the ultrasound diagnosis apparatus 100 automatically detects and displays a long bone from volume data without requiring a user to repeat an operation of directly scanning an object, for detecting the long bone. Accordingly, a user can conveniently and accurately diagnose an object, and thus can solve a problem in which an error of a diagnosis result occurs due to a dependency on the expertise of a user.

FIG. 5 is a diagram illustrating an example in which a measured length of a long bone is displayed on the screen 400 of the ultrasound diagnosis apparatus 100, in association with an embodiment of the present invention.

In FIG. 5, the ultrasound diagnosis apparatus 100 detects a long bone, and before displaying the long bone volume data 441 in the fourth area 440, the ultrasound diagnosis apparatus 100 measures a length of the long bone. Subsequently, the ultrasound diagnosis apparatus 100 may display the measured length of the long bone in a predetermined area 450 of the screen 400.

According to the embodiment of FIG. 5, even without directly measuring a length of a long bone by using a gradation, the ultrasound diagnosis apparatus 100 can accurately measure a length of a detected long bone and provide the measured length to a user. Accordingly, the ultrasound diagnosis apparatus 100 can accurately measure a change in length of a long bone, according to skills of a user.

The embodiments of the present invention can be written as computer programs and can be implemented in general-use digital computers that execute the programs using a computer readable recording medium. Also, a structure of data used in the aforementioned embodiments may be recorded in computer-readable recording media through various members. Program storage devices usable for describing a storage device including executable computer codes for performing various methods of the present invention should not be understood as including transitory targets like carrier waves or signals. Examples of the computer readable recording medium include magnetic storage media (e.g., ROM, floppy disks, hard disks, etc.) and optical recording media (e.g., CD-ROMs, or DVDs).

According to the above-described method and apparatus, a long bone of a fetus is measured from volume data, and the measured result is displayed to a user, thus enabling the user to simply and accurately diagnose the long bone of the fetus.

Moreover, a 3D-rendered ultrasound image is provided to the user, and thus, the user can diagnose an object by using a high-quality image of the long bone.

## Claims

1. An ultrasound diagnosis method comprising:
deciding a reference plane for volume data acquired from an object, wherein the reference plane which is obtained by cutting the volume data along at least one selected from an axial plane, a sagittal plane, and a coronal plane;
detecting a long bone included in the volume data on a basis of at least one of pixel values and shapes of a plurality of areas included in the reference plane;
extracting long bone volume data including the detected long bone from the volume data; and
displaying the extracted long bone volume data.

2. The ultrasound diagnosis method of claim 1, further comprising:
setting a reference point for the extracted long bone volume data; and
rotating the extracted long bone volume data with respect to the reference point such that a length axis of the long bone volume data is toward a predetermined direction.

3. The ultrasound diagnosis method of claim 1, further comprising measuring a length of the detected long bone and displaying the measured length.

4. The ultrasound diagnosis method of claim 1, wherein the extracting of long bone volume data includes extracting volume data having a predetermined thickness from a border of the detected long bone.

5. The ultrasound diagnosis method of claim 1, wherein,
the object includes a body of a pregnant woman, and
the volume data includes volume data of a fetus inside the pregnant woman.

6. The ultrasound diagnosis method of claim 1, wherein the long bone includes at least one of a humerus, a radius, an ulna, a femur, a fibula, and a tibia.

7. An ultrasound diagnosis apparatus (100) comprising:
a volume data acquirer (110) that scans an object to acquire volume data;
a reference plane decider (120) that decides a reference plane for volume data acquired from an object, wherein the reference plane is obtained by cutting the volume data along at least one selected from an axial plane, a sagittal plane, and a coronal plane;
a long bone detector (130) that detects a long bone included in the volume data on a basis of at least one of pixel values and shapes of a plurality of areas included in the reference plane;
an image processor (140) that extracts long bone volume data including the detected long bone from the volume data; and
a display (150) that displays the extracted long bone volume data.

8. The ultrasound diagnosis apparatus (100) of claim 7, wherein the image processor (140) sets a reference point for the extracted long bone volume data, and rotates the extracted long bone volume data with respect to the reference point such that a length axis of the long bone volume data is toward a predetermined direction.

9. The ultrasound diagnosis apparatus (100) of claim 7, wherein,
the image processor (140) measures a length of the detected long bone, and
the display (150) displays the measured length.

10. The ultrasound diagnosis apparatus of claim 7, wherein the image processor (140) extracts volume data having a predetermined thickness from a border of the detected long bone.

11. A non-transitory computer-readable record medium storing a program, which, when executed by a computer, performs the method of claim 1.

## Patentansprüche

1. Verfahren zur Ultraschalldiagnose, welches Folgendes aufweist:
Festlegen einer Referenzebene für Volumendaten, die von einem Objekt erlangt werden, wobei die Referenzebene durch Schneiden der Volumendaten entlang wenigstens einer aus einer Axialebene, einer Sagittalebene und einer Koronarebene erlangt wird;
Detektieren eines Röhrenknochens, der in den Volumendaten enthalten ist, auf einer Basis von wenigstens einem von Pixelwerten und Formen einer Vielzahl von Flächen, die in der Referenzebene enthalten sind;
Extrahieren von Röhrenknochen-Volumendaten, die den detektierten Röhrenknochen enthalten, aus den Volumendaten; und
Anzeigen der extrahierten Röhrenknochenvolumendaten.

2. Verfahren zur Ultraschalldiagnose nach Anspruch 1, welches des Weiteren Folgendes aufweist:
Festlegen eines Referenzpunkts für die extrahierten Röhrenknochenvolumendaten; und
Rotieren der extrahierten Röhrenknochenvolumendaten unter Bezugnahme auf den Referenzpunkt, so dass eine Längsachse der Röhrenknochenvolumendaten in eine bestimmte Richtung zeigt.

3. Verfahren zur Ultraschalldiagnose nach Anspruch 1, welches des Weiteren das Messen einer Länge des detektierten Röhrenknochens und das Anzeigen der gemessenen Länge aufweist.

4. Verfahren zur Ultraschalldiagnose nach Anspruch 1, wobei das Extrahieren der Röhrenknochenvolumendaten das Extrahieren von Volumendaten, die eine bestimmte Dicke aufweisen, von einem Rand des detektierten Röhrenknochens beinhaltet.

5. Verfahren zur Ultraschalldiagnose nach Anspruch 1, wobei
das Objekt einen Körper einer schwangeren Frau beinhaltet; und
die Volumendaten Volumendaten eines Fötus innerhalb der schwangeren Frau beinhalten.

6. Verfahren zur Ultraschalldiagnose nach Anspruch 1, wobei der Röhrenknochen wenigstens einen eines Humerus, eines Radius, einer Ulna, eines Femur, einer Fibula und einer Tibia beinhaltet.

7. Vorrichtung (100) zur Ultraschalldiagnose, welche Folgendes aufweist:
eine Volumendatenerlangungseinrichtung (110), das ein Objekt scannt, um Volumendaten zu erlangen;
eine Referenzebenenfestlegeeinrichtung (120), die eine Referenzebene für Volumendaten, die von einem Objekt erlangt werden, festlegt, wobei die Referenzebene durch Schneiden der Volumendaten entlang wenigstens einer, die aus einer Axialebene, einer Sagittalebene und einer Koronarebene ausgewählt ist, erlangt wird;
eine Röhrenknochendetektionseinrichtung (130), die einen Röhrenknochen detektiert, der in den Volumendaten enthalten ist, auf der Basis von wenigstens einem von Pixelwerten und Formen einer Vielzahl von Flächen, die in der Referenzebene enthalten sind;
eine Bildverarbeitungseinrichtung (140), die Röhrenknochenvolumendaten aus den Volumendaten extrahiert, die den detektierten Röhrenknochen beinhalten; und
eine Anzeige (150), welche die extrahierten Röhrenknochenvolumendaten anzeigt.

8. Vorrichtung (100) zur Ultraschalldiagnose nach Anspruch 7, wobei die Bildverarbeitungseinrichtung (140) einen Referenzpunkt für die extrahierten Röhrenknochenvolumendaten festlegt und die extrahierten Röhrenknochenvolumendaten unter Bezugnahme auf den Referenzpunkt derart rotiert, dass eine Längsachse der Röhrenknochenvolumendaten in eine bestimmte Richtung zeigt.

9. Vorrichtung (100) zur Ultraschalldiagnose nach Anspruch 7, wobei
die Bildverarbeitungsvorrichtung (140) eine Länge des detektierten Röhrenknochens misst und die Anzeige (150) die gemessene Länge anzeigt.

10. Vorrichtung zur Ultraschalldiagnose nach Anspruch 7, wobei die Bildverarbeitungseinrichtung (140) Volumendaten, die eine bestimmte Dicke aufweisen, von einem Rand des detektierten Röhrenknochens extrahiert.

11. Nicht flüchtiges, computerlesbares Speichermedium, das ein Programm speichert, das, wenn es von einem Computer ausgeführt wird, das Verfahren nach Anspruch 1 durchführt.

## Revendications

1. Procédé de diagnostic à ultrasons comprenant :
la décision d'un plan de référence pour des données de volume acquises à partir d'un objet, dans lequel le plan de référence qui est obtenu en coupant les données de volume le long d'au moins un plan choisi parmi un plan axial, un plan sagittal et un plan coronal ;
la détection d'un os long inclus dans les données de volume sur la base d'au moins une des valeurs et formes de pixels d'une pluralité de zones incluses dans le plan de référence ;
l'extraction de données de volume d'os long comprenant l'os long détecté à partir des données de volume ; et
l'affichage des données de volume d'os long extraites.

2. Procédé de diagnostic à ultrasons selon la revendication 1, comprenant en outre :
la définition d'un point de référence pour les données de volume d'os long extraites ; et
la rotation des données de volume d'os long extraites par rapport au point de référence de telle sorte qu'un axe de longueur des données de volume d'os long est dans une direction prédéterminée.

3. Procédé de diagnostic à ultrasons selon la revendication 1, comprenant en outre la mesure d'une longueur de l'os long détecté et l'affichage de la longueur mesurée.

4. Procédé de diagnostic à ultrasons selon la revendication 1, dans lequel l'extraction de données de volume d'os long comprend l'extraction de données de volume ayant une épaisseur prédéterminée à partir d'un bord de l'os long détecté.

5. Procédé de diagnostic à ultrasons selon la revendication 1, dans lequel, l'objet comprend un corps de femme enceinte, et
les données de volume incluent les données de volume d'un foetus à l'intérieur de la femme enceinte.

6. Procédé de diagnostic à ultrasons selon la revendication 1, dans lequel l'os long comprend au moins l'un parmi un humérus, un radius, un cubitus, un fémur, un péroné et un tibia.

7. Aappareil de diagnostic à ultrasons (100) comprenant :
un acquéreur de données de volume (110) qui balaye un objet pour acquérir des données de volume ;
un décideur de plan de référence (120) qui détermine un plan de référence pour des données de volume acquises à partir d'un objet, dans lequel le plan de référence est obtenu en coupant les données de volume le long d'au moins un plan choisi parmi un plan axial, un plan sagittal, et un plan coronal ;
un détecteur d'os long (130) qui détecte un os long inclus dans les données de volume sur la base d'au moins une des valeurs et formes de pixels d'une pluralité de zones incluses dans le plan de référence ;
un processeur d'image (140) qui extrait des données de volume d'os long comprenant l'os long détecté à partir des données de volume ; et
un écran (150) qui affiche les données de volume d'os long extraites.

8. Appareil de diagnostic à ultrasons (100) selon la revendication 7, dans lequel le processeur d'image (140) définit un point de référence pour les données de volume d'os long extraites, et fait pivoter les données de volume d'os long extraites par rapport au point de référence de telle sorte qu'un axe de longueur des données de volume d'os long est dans une direction prédéterminée.

9. Appareil de diagnostic à ultrasons (100) selon la revendication 7, dans lequel, le processeur d'image (140) mesure une longueur de l'os long détecté, et l'écran (150) affiche la longueur mesurée.

10. Appareil de diagnostic à ultrasons selon la revendication 7, dans lequel le processeur d'image (140) extrait des données de volume ayant une épaisseur prédéterminée à partir d'un bord de l'os long détecté.

11. Support d'enregistrement lisible par ordinateur non transitoire stockant un programme, qui, lorsqu'il est exécuté par un ordinateur, effectue le procédé selon la revendication 1.
